Europäisches Patentamt

European Patent Office

Office européen des brevets

(11) Publication number: **0 335 023**
**A1**

(12) **EUROPEAN PATENT APPLICATION**

(21) Application number: 88302907.6

(51) Int. Cl.⁴: **C12N 9/58**

(22) Date of filing: 31.03.88

| | |
|---|---|
| The microorganisms have been deposited with the Culture Collection of the Commonwealth Mycological Institute under numbers CMI CC 319793 - 319794 - 319795 - 319796 - 319797 - 319718. | (71) Applicant: **AGRICULTURAL GENETICS COMPANY LIMITED** **Unit 154/155 Cambridge Science Park Milton Road** **Cambridge CB4 4BH(GB)** |
| (43) Date of publication of application: **04.10.89 Bulletin 89/40** | (72) Inventor: **Charnley, Anthony Keith** **41 Leigh Park Road** **Bradford upon Avon, Wiltshire(GB)** Inventor: **Cooper, Richard Martin** **Whittington, Lower Kingsdown Road, Kingsdown, Bath, Avon(GB)** Inventor: **St Leger, Raymond John, Boyce Inst. for Plant Research, Cornell Univ. Tower Road, Ithaca, New York(US)** |
| (84) Designated Contracting States: **AT BE CH DE ES FR GB GR IT LI LU NL SE** | |
| | (74) Representative: **Lewin, John Harvey et al** **ELKINGTON AND FIFE Beacon House 113 Kingsway** **London WC2B 6PP(GB)** |

(54) **Fungal enzymes.**

(57) The invention provides an enzyme preparation comprising at least one protease derived from an entomopathogenic fungus of one of the following genera: Metarhizium, Beauvaria, Verticillium, and Aschersonia, and characterised by the following properties:

    (a) substantially no activity against hide protein azure, locust cuticle and elastin;

    (b) specificity for Bz-Phe-Val-Arg-NA;

    (C) not inhibited by soybean trypsin inhibitor.

## FUNGAL ENZYMES

This invention relates to fungal enzymes and more particularly to highly specific proteases produced by entomopathogenic fungi.

The entomopathogenic fungus Metarhizium anisopliae (isolate ME1) has been shown to produce a mixture of extracellular proteases (R.J. St. Leger, A.K. Charnley and R.M. Cooper, Characterization of cuticle-degrading proteases produced by the entomopathogen Metarhizium anisopliae ME1, Archives of Biochemistry and Biophysics (1987) 253, 221-232). The proteases have been resolved into two major components: chymoelastase (designated Pr1) and a trypsin-like protease (designated Pr2); isoenzymes of Pr2 are present.

Pr1 has a broad substrate specificity but Pr2 has a much narrower substrate range. Although Pr2 rapidly hydrolyses casein and synthetic substrates containing arginine or lysine it has little or no activity against host insect cuticle, elastin, or synthetic substrates for chymotrypsin and elastase. Specific active site inhibitors confirmed the similarities between Pr2 and trypsin.

A further 8 isolates of the entomopathogenic fungi M. anisopliae (4 isolates), Beauvaria bassiana, Verticillium lecanii (2 isolates), and Aschersonia aleyrodis have been screened for extracellular proteases. All the isolates produce Pr1 and Pr2 proteases but, surprisingly, the Pr2 proteases of 6 of these isolates have a very narrow substrate specificity when compared to the previously described Pr2 protease of M. anisopliae (ME1).

The Pr2 proteases of M. anisopliae isolates ME1, RS549 and RS23 are active against hide protein azure but not against locust cuticle and elastin (table 1). In contrast, Pr2 proteases from the other 6 isolates are inactive against all three proteins. Further evidence of the restricted substrate specificity of the Pr2 proteases from the other 6 isolates was obtained from studies on artificial substrates (nitroanilide derivatives of peptides). Pr2 enzymes from ME1, RS549 and RS23 hydrolysed a series of substrates Bz-AA-AA-Arg-NA thus demonstrating little discrimination in their secondary subsite specificities (table 2, see below). In contrast, the Pr2 proteases from the other 6 isolates showed a specificity for Bz-Phe-Val-Arg-NA. Thus these enzymes require extremely specific substrate sequences and as such they represent a previously unreported class of fungal protease. The only other enzymes reported with similar restricted specifities are certain thrombins such as Thrombocytin (from Bothrops atox venom). Thrombocytin rapidly cleaves Bz-Phe-Val-Arg-NA with minor activity against Bz-Pro-Phe-Arg-NA.

Further differences between the Pr2 proteases from ME1, RS549 and RS23, and those from the other 6 isolates were revealed by inhibitor studies (table 3, see below). Pr2 proteases from ME1, RS549 and RS23, like thrombocytin, are inhibited by soyabean trypsin inhibitor whereas Pr2 proteases from the other 6 isolates (except A. aleyrodis which was not tested) are not inhibited.

Such highly specific proteases may have useful pharmaceutical, agricultural or industrial applications.

The present invention thus provides an enzyme preparation comprising at least one protease derived from an entomopathogenic fungus of one of the following genera: Metarhizium, Beauvaria, Verticillium, and Aschersonia, and characterised by the following properties:

    (a) substantially no activity against hide protein azure, locust cuticle and elastin;

    (b) specificity for Bz-Phe-Val-Arg-NA;

    (c) not inhibited by soybean trypsin inhibitor.

In a preferred aspect, the protease is derived from one of the following species: Metarhizium anisopliae, Beauvaria bassiana, Verticillium lecanii, and Aschersonia aleyrodis.

In a particularly preferred aspect, the protease is derived from one of the following strains, or a derivative or mutant thereof capable of producing said protease. These strains have been deposited at the Commonwealth Mycological Institute (CMI), Ferry Lane, Kew, Richmond, Surrey, TW9 3AF, U.K. on 12 October 1987 and have the following culture collection numbers:

| Microorganism | | | | | CMI CC Number |
|---|---|---|---|---|---|
| Metarhizium anisopliae var major RS 324 | | | | | 319793 |
| " | " | " | " | RS 298 | 319794 |
| Verticillium lecanii | 1 | | | | 319795 |
| " | " | 2 | | | 319796 |
| Beauveria bassiana | | | | | 319797 |
| Aschersonia aleyrodis | | | | | 319798 |

The characteristics of the deposited strains referred to above essentially correspond to the description of typed strains which have been published.

The invention is illustrated by the following Examples

## Abbreviations

| | |
|---|---|
| Bz-AA-AA-Arg-NA: | N-benzoyl-L-aminoacid-L-aminoacid-L-arginine-p-nitroanilide. |
| Bz-Phe-Val-Arg-NA: | N-benzoyl-L-phenylalanine-L-valine-L-arginine-p-nitroanilide. |
| Bz-Val-Gly-Arg-NA: | N-benzoyl-L-valine-glycine-L-arginine-p-nitroanilide. |
| Bz-Pro-Phe-Arg-NA: | N-benzoyl-L-proline-L-phenylalanine-L-arginine-p-nitroanilide. |
| CBZ-Gly-Pro-Arg-NA: | N-carbobenzoxy-glycine-L-proline-L-arginine-p-nitroanilide. |
| Bz-Arg-NA: | N-benzoyl-L-arginine-p-nitroanilide. |
| D-Val-Leu-Lys-NA: | D-valine-L-leucine-L-lysine-p-nitroanilide. |
| Suc-(Ala)$_2$-Pro-Phe-NA: | N-succinyl-L-alanine-L-alanine-L-proline-L-phenylalanine-p-nitroanilide. |
| Suc-Phe-Leu-Phe-NA: | N-succinyl-L-phenylalanine-L-leucine-L-phenylalanine-p-nitroanilide. |
| Suc-(Ala)$_2$-Pro-Leu-NA: | N-succinyl-L-alanine-L-alanine-L-proline-L-leucine-p-nitroanilide. |

Suc-(Ala)$_2$-Pro-Abu-NA:  N-succinyl-L-alanine-L-proline-L-amino-n-butyric acid-p-nitroanilide.

Suc-(Ala)$_2$-Val-Ala-NA:  N-succinyl-L-alanine-L-alanine-L-valine-L-alanine-p-nitroanilide.

Ac-(Ala)$_3$-NA:  N-acetyl-L-alanine-L-alanine-L-alanine-p-nitroanilide.

PMSF:  Phenylmethylsulphonyl fluoride.

Tos-Lys-CH$_2$Cl:  N$\alpha$-p-tosyl-L-lysine-chloromethylketone.

Tos-Phe-CH$_2$Cl:  N-tosyl-L-phenylalanine-chloromethylketone.

BOC-Gly-Leu-Phe-CH$_2$Cl:  N-tert-butoxycarbonyl-glycine-L-leucine-L-phenylalanine-chloromethylketone.

EXAMPLES

1. Producer Organisms

Metarhizium anisopliae var. major RS 324
M. anisopliae var. major RS 298
Verticillium lecanii 1
V. lecanii 2
Beauveria bassiana
Aschersonia aleyrodis

2. Growth conditions

The Metarhizium and Verticillium isolates were maintained on Sabouraud's Dextrose agar at 23°C (V. lecanii) or 27°C (M. anisopliae). The B. bassiana isolate was maintained at Czapek-Dox medium at 27°C. The A. aleyrodis isolate was maintained on Maltose agar at 23°C.

Fungi were grown in 100 ml of growth medium in 250 ml Erlenmeyer flasks. The composition of the growth medium was as follows:
1% w/v carbon source
0.1% w/v KH$_2$PO$_4$
0.05% w/v MgSO$_4$
0.05M 4-morpholine ethanesulphonic acid pH6.0
0.2 $\mu$g/ml FeSO$_4$.7H$_2$O
1.0 $\mu$g/ml ZnSO$_4$.7H$_2$O
0.2 $\mu$g/ml NaMoO$_4$.2H$_2$O
0.02 $\mu$g/ml CuSO$_4$.5H$_2$O
0.02 $\mu$g/ml MnCl$_2$.4H$_2$O

The following polymeric, non-repressing carbon sources can be used: locust cuticle, bovine serum albumen, laminarin, cellulose, elastin, collagen. When cellulose is used, the growth medium must be supplemented with 0.2% w/v NaNO$_3$ as a nitrogen source.

Insoluble carbon sources were added to previously sterilized basal media (121°C, 15 min) and autoclaved for 5 min at 115° C. Soluble carbon sources were autoclaved in basal media for 20 min at 115° C.

After inoculation with 3 x 10⁶ spores taken from 7-to 12- day agar plates the flasks were shaken on a rotary incubator (150 rev/min) at 23° C (V. lecanii, A. aleyrodis) or 27.5° C (M. anisopliae and B. bassiana).

3. Purification of enzymes

Cultures grown for 5d (14d with A. aleyrodis) in 1% locust cuticle basal salts media (200 ml in 500 ml conical flasks rotated at 150 rpm) were clarified by filtration (through Whatman 1 paper) and centrifugation (8,000g, 15 min at 3° C). The solid material (cuticle and fungal bodies) was washed in 0.1 M K phosphate buffer (ca. 3% w/v, wet wt) to remove electrostatically bound enzymes, filtered and centrifuged. This filtrate and the original culture filtrate were dialysed separately (4° C, 12h vs 2 x 300 vol dist H₂O, pH 6.0), concentrated (vs polyethylene glycol M.W. 20,000) and combined (final volume 50 ml) before preparative isoelectric focussing (IEF) as described below. The pH of fractions was determined (the gradient was discontinuous above pH 10.0 so pI values above 10.0 were approximations) and fractions were assayed for enzymatic activity following dialysis against 0.2 M KCl (4° C, 12h) and then distilled water (4° C, 12h).

Preparative IEF (pH 3.5 - 10.0) was performed with a 440 ml (LKB 8100) column. Narrow range IEF was performed in a 110 ml (LKB 8101) column. The procedures were described in the manufacturer's handbook. Enzyme activities and protein levels in the eluate were determined after removal of ampholytes by exhaustive dialysis against 0.2M KCl (14h, 4° C, 300 vol) and distilled water (12h, 4° C, 300 vol) at pH 6.0.

4. Characterization of enzymes

a. Assays of enzyme activity

Non-specific protease activity was measured with hide protein azure. 5 ml bottles were charged with 20 mg of substrate, 4 ml of Britton Robinson buffer of required pH, and 1 ml of enzyme. The bottles were stoppered and fastened to a revolving disk. After incubation at 30° C, reactions were terminated by the addition of trichloroacetic acid (0.25 ml, 500 g/litre). After centrifugation (5,000 g, 10 min) absorbance was measured at 595 nm. Activities are expressed as μg trypsin equivalents/ml/hr calculated from a standard curve of trypsin activity against hide protein azure. The results are shown in table 1.

Elastase activity was estimated in a reaction mixture containing 4 ml Tris buffer (0.05M, pH 8.0), 1 ml of enzyme solution, and elastin - Congo Red (1 mg ml⁻¹ protein). After appropriate time intervals at 30° C the mixtures were centrifuged (5000g, 10 min) and the absorbance was determined at 450 nm. One elastolytic unit was defined as the amount of enzyme which solubilized 1 mg of elastin in 30 min.

Activity against purified proteins was assayed as follows: 0.5 ml of enzyme solution was incubated with 1.0 ml of 0.05M Tris buffer (pH 8.0) containing 2.5 mg ml⁻¹ protein. After appropriate periods of incubation at 30° C the reaction was terminated by adding 2 ml of 10% trichloroacetic acid and the tubes were allowed to stand for 1 hour. The residue of undigested protein was removed by filtration through Whatman No. 50 filter paper and the absorbance of the filtrate was read at 280 nm. Enzyme activities are expressed as μg tyrosine equivalents min⁻¹ ml⁻¹. Enzymic release of free α-amino groups from proteins was determined by a ninhydrin assay.

Activity versus locust cuticle was assayed as follows:

30 mg of ground locust cuticle (prepared using aqueous solutions of 1% potassium tetraborate) was vigorously shaken at 30° C with 2 ml of enzyme, 4 ml of Britton-Robinson buffer at pH 5.0 and 0.05 ml of toluene. Samples (0.05 ml) were taken at intervals up to 24 hours incubation. Release of amino acids due to proteolytic activity was determined with ninhydrin.

The results are shown in Table 1.

Activity against nitroanilide substrates was assayed in reaction mixtures containing 0.1 ml enzyme solution and 1.8 ml Britton-Robinson buffer pH 8.0. After 3 min at 23° C 0.1 ml substrate (in dimethylsulphoxide) was added and the reaction followed at 410 nm. Results are expressed as μmol nitroanilide released ml⁻¹ min⁻¹. An extinction coefficient of 8800 at 410 nm was used. The results are shown in Table 2.

All controls contained autoclaved enzyme (121° C for 30 min) in place of unheated enzyme solutions.

b. Determination of molecular weight

Molecular weights were determined by SDS- polyacrylamide gel electrophoresis in neutral or alkali-gel systems and by gel filtration on Sephadex G-100 Fine in 0.15 M phosphate buffer. The results are shown in table 4.

Table 1

| Activity of Pr2 proteases towards proteins | | | |
|---|---|---|---|
| | Protein | | |
| | Locust cuticle | Elastin | Hide protein azure |
| M. anisopliae ME 1 * | + | - | + |
| " RS 23 * | - | - | + |
| " RS 549 * | - | - | + |
| " RS 298 | - | - | - |
| " RS 324 | - | - | - |
| V. lecanii 1 | - | - | - |
| " 2 | - | - | - |
| B. bassiana | - | - | - |
| A. aleyrodis | - | - | - |
| + activity detected | | | |
| - no activity detected | | | |

* Comparison, not according to the invention

6

## Table 2    Substrate specificities of Pr2 proteases

|  | M.anisopliae | | | | B.bassiana | V.lecanii | V.lecanii | A.aleyrodis |
|---|---|---|---|---|---|---|---|---|
|  | RS23 | RS549 | RS298 | RS324 | | 1 | 2 | |
|  | * | * | | | | | | |
| **Substrates** | | | | | | | | |
| Bz-Phe-Val-Arg-NA | 66.7 | 66.7 | 100 | 100 | 100 | 100 | 100 | 100 |
| Bz-Val-Gly-Arg-NA | 83.3 | 55.6 | 7.6 | 0.1 | 0 | 0 | 0 | 0.1 |
| Bz-Pro-Phe-Arg-NA | 12.5 | 33.3 | 0.1 | 0.8 | 5.6 | 0 | 1.94 | 0.1 |
| CBZ-Gly-Pro-Arg-NA | 100 | 100 | 10.0 | 0.8 | 0.21 | 1.5 | 0.31 | 0.1 |
| Bz-Arg-NA | 0.7 | 0.1 | 0 | 0 | 0.1 | 0 | 0 | 0.1 |
| D-Val-Leu-Lys-NA | 54.2 | 0.1 | 0.5 | 0.3 | 0 | 0 | 0.78 | 0.1 |
|  | (1.27) | (8.13) | (1.06) | (0.56) | (5.25) | (2.01) | (2.31) | (0.13) |

\* Comparison, not according to the invention

Protease preparations after IEF were assayed at 23°C in Britton-Robinson buffer (pH 8.0), 4% (v/v) dimethylsulphoxide, 0.06 mM substrate. Activities are expressed as % of maximum activity. Absolute values ($\mu$Mols NA $min^{-1}ml^{-1}$ per mg protein) corresponding to 100% are given in parenthesis. The enzymes showed no activity or trace activity (<0.1%) against Suc-$(Ala)_2$-Pro-Phe-NA, Suc-Phe-Leu-Phe-NA, Suc-$(Ala)_2$-Pro-Leu-NA, Suc-$(Ala)_2$-Pro-Abu-NA, Suc-$(Ala)_2$-Val-Ala-NA or Ac-$(Ala)_3$-NA. _M. anisopliae_ ME1 protease Pr2 has a similar specificity to RS 23 and RS 549.

EP 0 335 023 A1

Table 3

| The effect of inhibitors on Pr2 activity | | | | | | | | |
|---|---|---|---|---|---|---|---|---|
| | M.anisopliae | | | | | B.bassiana | V.lecanii | |
| | ME1 | RS23 | RS549 | RS298 | RS324 | | 1 | 2 |
| PMSF (0.2mM) | 7.0 | 27.6 | 33.3 | 2.5 | 1.7 | 0.1 | 0.1 | 0.1 |
| TOS-Lys-CH₂Cl (0.05mM) | 46.6 | 35.7 | 14.7 | 100 | 100 | 87.5 | 87.5 | 100 |
| Leupeptin (50μml⁻¹) | 5.6 | 0.12 | 12.27 | 66.7 | 0.34 | 20.0 | 0.4 | 68.8 |
| Antipain (50μml⁻¹) | - | 0.1 | 13.3 | 95.0 | 62.5 | 60.0 | 41.7 | 94.4 |
| Soya bean trypsin inhibitor (50μml⁻¹) | 0.1 | 0.1 | 26.7 | 100 | 100 | 89.1 | 100 | 100 |
| N-Ethylmaleimide (1mM) | 97.0 | 100 | 100 | 100 | 100 | 100 | 100 | 100 |
| TOS-Phe-CH₂Cl (0.05mM) | 100 | 100 | 100 | 100 | 100 | 100 | 100 | 100 |
| BOC-Gly-Leu-Phe-CH₂Cl (0.05 mM) | 100 | 100 | 100 | 100 | 100 | 100 | 100 | 100 |
| Turkey egg white inhibitor (50μml⁻¹) | 100 | 100 | 100 | 100 | 100 | 100 | 100 | 100 |
| Hirudin (20μml⁻¹) | - | 100 | 100 | 100 | 100 | 100 | 100 | 100 |

\* Comparison, not according to the invention

Note:

The enzymes were preincubated with each inhibitor in Britton-Robinson buffer (pH8.0) at 25°C for 15 min before assaying with Bz-Phe-Val-Arg-NA. Activities are expressed as a % of the control activity.

Table 4

| Physical parameters of Pr2 proteases | |
|---|---|
| | Isoelectric point (pI) |
| M. anisopliae ME 1 \* | 4.4 |
| " RS 23 \* | 5.0 |
| " RS 549 \* | 6.0 |
| " RS 298 | 5.8 |
| " RS 324 | 5.8 |
| V. lecanii 1 | 5.6 |
| " 2 | 5.6 |
| B. bassiana | 7.4 |
| A. aleyrodis | 4.8 |
| Molecular weights of all enzymes:25000 - 30000. | |
| Optimum pH for activity :8.0 (except ME1 which is 9.0). | |
| Optimum pH for stability :6.0 - 9.0. | |
| Temperature stability : stable at temperatures up to 50°C (10 min at pH 8.0). | |

\* Comparison, not according to the invention.

**Claims**

1. An enzyme preparation comprising at least one protease derived from an entomopathogenic fungus of one of the following genera: Metarhizium, Beauvaria, Verticillium, and Aschersonia, and characterised by the following properties:
   (a) substantially no activity against hide protein azure, locust cuticle and elastin;
   (b) specificity for Bz-Phe-Val-Arg-NA.
   (c) not inhibited by soybean trypsin inhibitor.

2. An enzyme preparation according to Claim 1, in which the protease is derived from one of the following species: Metarhizium anisopliae, Beauvaria bassiana, Verticillium lecanii, and Aschersonia aleyrodis.

3. An enzyme preparation according to Claim 1 or 2, in which the protease is derived from one of the following strains, or a derivative or mutant thereof capable of producing said protease: CMI 319793, CMI 319794, CMI 319795, CMI 319796, CMI 319797 and CMI 319798.

4. An enzyme preparation according to any of Claims 1 to 3, in which the protease has a molecular weight of 25000 to 30000, an optimum pH for activity of about 8.0, an optimum pH for stability of 6.0 to 9.0, and is stable at temperatures up to 50°C.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,A | ARCHIVES OF BIOCHEMISTRY AND BIOPHYSICS, vol. 253, no. 1, 15th February 1987, pages 221-232, Academic Press, Inc.; R.J.ST. LEGER et al.: "Characterization of cuticle-degrading proteases produced by the entomopathogen Metarhizium anisopliae" --- | | C 12 N 9/58 |
| A | JOURNAL OF INVERTEBRATE PATHOLOGY, vol. 47, no. 2, 1986, pages 167-177, Academic Press, Inc.; R.J.ST. LEGER et al.: "Cuticle-degrading enzymes of entomopathogenic fungi: cuticle degradation in vitro by enzymes from entomopathogens" --- | | |
| A | JOURNAL OF INVERTEBRATE PATHOLOGY, vol. 48, 1986, pages 85-95, Academic Press, Inc.; R.J.ST. LEGER et al.: "Cuticle-degrading enzymes of entomopathogenic fungi: synthesis in culture on cuticle" --- | | TECHNICAL FIELDS SEARCHED (Int. Cl.4) |
| A | APPLIED AND ENVIRONMENTAL MICROBIOLOGY, vol. 53, no. 7, July 1987, pages 1679-1684, American Society for Microbiology; M.J. BIDOCHKA et al.: "Purification and properties of an extracellular protease produced by the entomopathogenic fungus Beauveria bassiana" --- -/- | | C 12 N |

The present search report has been drawn up for all claims

| Place of search | Date of completion of the search | Examiner |
|---|---|---|
| THE HAGUE | 01-12-1988 | DESCAMPS J.A. |

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | JOURNAL OF INVERTEBRATE PATHOLOGY, vol. 35, no. 3, 1980, pages 304-310, Academic Press, Inc.; M. KUCERA: "Proteases from the fungus Metarhizium anisopliae toxic for Galleria mellonella larvae" | | |
| A | US-A-2 936 265 (A.R. WHITEHILL et al.) | | |
| A | US-A-2 927 060 (K. ORINGER) | | |

TECHNICAL FIELDS
SEARCHED (Int. Cl.4)

The present search report has been drawn up for all claims

| Place of search | Date of completion of the search | Examiner |
|---|---|---|
| THE HAGUE | 01-12-1988 | DESCAMPS J.A. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P0401)